# EUROPEAN PATENT APPLICATION

(11) **EP 4 342 424 A1**
(43) Date of publication of application: **27.03.2024**
(21) Application number: 22885292.7
(22) Date of filing: 11.08.2022
(51) Int. Cl.: A61F 2/24

(54) **IMPLANT DELIVERY DEVICE AND IMPLANT DELIVERY SYSTEM**

(30) Priority: 28.10.2021 CN 202111265129
(71) Applicant: Shanghai Trulive Medtech Co., Ltd., Shanghai 201206 (CN); Jiangsu Trulive Medtech Co., Ltd., Nantong, Jiangsu 226400 (CN)
(72) Inventor: ZHANG, Wei, Shanghai 201206 (CN); XU, Haoran, Shanghai 201206 (CN); ZHAO, Jing, Shanghai 201206 (CN); WEI, Yongqiang, Shanghai 201206 (CN)
(74) Representative: Nordmeyer, Philipp Werner
(86) International application number: PCT/CN2022/111799
(87) International publication number: WO 2023/071392

(57) **Abstract**

An implant delivery device and an implant delivery system. The implant delivery device is applied to a catheter assembly (70) including a first inner tube (71) and a second inner tube (72). The implant delivery device includes two power conversion assemblies (10) which are sequentially arranged in the direction of a baseline (H), each of which includes a master member (11) and a slave member (12) in engagement with the master member (11). The slave member (12) of one of the two power conversion assemblies (10) is adapted for connection with the first inner tube (71), and the slave member (12) of the other of the two power conversion assemblies (10) is adapted for connection with the second inner tube (72). The power conversion assemblies (10) are configured to convert a rotational motion of the master members (11) to a linear motion of the slave members (12) in the direction of the baseline (H). Controlling movement of the first inner tube (71) and the second inner tube (72) respectively with the two power conversion assemblies (10) enables desirable independent movement of the first inner tube (71) and the second inner tube (72). Moreover, relative movement of the first inner tube (71) and the second inner tube (72) can be controlled in a desired style to allow a valve stent (80) to be released in two or more steps. This can facilitate precise release and positioning of the valve stent (80).

## Description

### TECHNICAL FIELD

The present invention relates to the field of medical devices and, in particular, to an implant delivery device and an implant delivery system.

### BACKGROUND

Heart valves are openable and closeable flap-like structures in the organs of humans and some animals. The human heart has four valves: the aortic valve connecting the left ventricle to the aorta; the pulmonary valve connecting the right ventricle to the pulmonary artery; the mitral valve connecting the left atrium to the left ventricle; and the tricuspid valve connecting the right atrium to the right ventricle. All these valves function like one-way valves which allow blood flow in the forward direction but prevent its backward flow.

With the development of social economy and the aging of population, the incidence of valvular heart disease has increased significantly. Studies have shown that this figure has reached up to 13.3% among those 75 years or older. At present, traditional surgical treatment remains the first choice for patients with severe valvular disease. However, for those with advanced ages, complications in multiple organs, a history of thoracotomy or poor heart functions, the traditional surgical approach is associated with high risk and high mortality or even precludes some patients.

Interventional valve implantation is a brand new minimally invasive valve replacement technique that has been developed abroad in recent years, which involves loading a valve stent in a delivery system and delivering it into a human body through a catheter. The prosthesis can functionally replace the patient's dysfunctional native valve and improve his/her cardiac condition. This technique is able to treat valvular disease, without surgically opening the chest or stopping the heartbeat, which may cause significant trauma to the patient.

The structure of the human heart is very complex. In particular, the structure of the mitral valve is more complex even than that of the aortic valve because the mitral annulus has an irregular shape and there are many chordae tendineae in the ventricular chamber, which pose great challenges to the implantation and positioning of a prosthetic valve. For transcatheter valve replacement (including transcatheter aortic valve replacement (TAVI), transcatheter mitral valve replacement (TMVR)), safe and effective operation of the delivery system used is one of the key factors for surgical success. Accordingly, delivery systems for this purpose are required to allow safe and accurate operation because inferior operating accuracy of such systems may lead to operators' erroneous operation during procedures, inaccurate stent positioning, or even severe paravalvular leakage/regurgitation in patients in serious cases.

At present, common delivery systems rely on handles to achieve valve stent release speed control. That is, a handle may be turned faster or slower to allow a valve stent to be released at a consequent higher or lower speed. Different clinical needs and different types of prosthetic valves require such a handle to be turned at various speeds. For a valve stent that can be easily positioned accurately, releasing it as fast as possible by manipulating a handle would be desirable because this can provide the advantages of precise positioning, rapid release and a shorter surgical time. However, for complex anatomies which pose significant challenges to valve stent positioning, frequent position adjustments would be necessary and, therefore, slow valve stent release would be desirable in order to increase the rate of success. Therefore, how to develop a handle capable of being turned at precisely controlled speeds to result in various release speeds required by different valve stent applications is a focus of attention of experts and scholars.

### SUMMARY OF THE INVENTION

It is an objective of the present invention to provide an implant delivery device and an implant delivery system, which overcome the problem of inaccurate positioning of a valve stent during release associated with conventional delivery devices.

To this end, in one aspect of the present invention, there is provided a valve implant delivery device applied to a catheter assembly having a first inner tube and a second inner tube. The first inner tube is inserted in the second inner tube. The implant delivery device includes:
two power conversion assemblies sequentially arranged in the direction of a baseline, each of the power conversion assemblies including a master member and a slave member in engagement with the master member, the slave member of one of the two power conversion assemblies adapted for attachment to the first inner tube, the slave member of the other of the two power conversion assemblies adapted for attachment to the second inner tube, the power conversion assemblies configured to convert rotational motion of the master members to linear motion of the respective slave members in the direction of the baseline.

Optionally, the master member may include a screw extending in the direction of the baseline, wherein the slave member is threadedly engaged with an external thread section of the screw.

Optionally, the slave member may define a first internal bore and a second internal bore, which both extend through the slave member in the direction of the baseline, the first internal bore having an internal thread section engageable with the external thread section of the screw, wherein the second internal bore of one of the two power conversion assemblies is adapted for fixed attachment to the first inner tube, and the second internal bore of the other of the two power conversion assemblies is adapted for fixed attachment to the second inner tube.

Optionally, the master member may include a gear having an axis of rotation perpendicular to the baseline, wherein the slave member includes a rack in engagement with the gear and extending in the direction of the baseline.

Optionally, the master member may include a worm extending in the direction of the baseline, wherein the slave member includes a worm gear in engagement with the worm and having a center axis perpendicular to the baseline.

Optionally, the implant delivery device may further include drive members which are coupled to the respective master members and adapted to drive the respective master members to rotate.

Optionally, the implant delivery device may further include gear assemblies each including a first gear and a second gear in engagement with the first gear, the first gear coaxially coupled to the respective master member, the second gear coaxially coupled to the respective drive member.

Optionally, both the first and second gears may be bevel gears, wherein an axis of rotation of the master member is perpendicular to an axis of rotation of the drive member.

Optionally, when the slave member drives the corresponding first/second inner tube to move at a speed higher than or equal to a predetermined threshold, a transmission ratio of the second gear to the first gear may be greater than 1. Alternatively, when the slave member drives the corresponding first/second inner tube to move at a speed lower than the predetermined threshold, the transmission ratio of the second gear to the first gear may be smaller than or equal to 1.

Optionally, the second gear may be fixedly coupled to the drive member through a coupling shaft so that the drive member is able to drive the second gear to rotate through the coupling shaft, the coupling shaft having a center axis coincident with an axis of rotation of the second gear, wherein the drive member defines a special-shaped hole, and the coupling shaft has a special-shaped shaft section matching the special-shaped hole in shape.

Optionally, the drive members may be coaxially coupled to the master members and each have an axis of rotation parallel to the baseline.

Optionally, the implant delivery device may further include a spacer oriented perpendicular to the baseline, the spacer disposed between the master members of the two power conversion assemblies, the spacer attached to ends of the master members opposite to those thereof coupled to the drive members.

In another aspect of the present invention, there is provided an implant delivery system including:
the implant delivery device as defined above; and
a catheter assembly including a first inner tube and a second inner tube, the first inner tube inserted in the second inner tube, the second inner tube attached to the slave member in one of the two power conversion assemblies, the second inner tube adapted to restrict radial expansion of a valve stent, the first inner tube attached to the slave member in the other of the two power conversion assemblies, the first inner tube adapted for attachment to a proximal end of the valve stent.

Optionally, the catheter assembly may further include a third inner tube inserted in the first inner tube.

In summary, the present invention provides an implant delivery device and an implant delivery system. The implant delivery device includes two power conversion assemblies which are sequentially arranged in the direction of a baseline, each of which includes a master member and a slave member in engagement with the master member. The slave member of one of the two power conversion assemblies is adapted for attachment to a first inner tube, and the slave member of the other of the two power conversion assemblies is adapted for attachment to a second inner tube. The power conversion assemblies are configured to convert rotational motion of the master members to linear motion of the slave members in the direction of the baseline. Controlling movement of the first and second inner tubes respectively with the two power conversion assemblies enables desirable independent movement of the first and second inner tubes without mutual interference. Moreover, relative movement of the first and second inner tubes can be controlled in a desired manner to allow a valve stent to be released in two or more steps. This can facilitate precise release and positioning of the stent without paravalvular leakage or regurgitation occurring in a patient. Compared with the prior art, the use of the power conversion assemblies according to the present invention decouples the power that drives movement of the first and second inner tubes from its original form that is provided by an external mechanism. This can avoid the device from having a complex structure and reduce its design and manufacturing costs.

### BRIEF DESCRIPTION OF THE DRAWINGS

Those of ordinary skill in the art would appreciate that the accompanying drawings are provided to facilitate a better understanding of the present invention and do not limit the scope thereof in any sense, in which:
Fig. 1 is a schematic illustration of an implant delivery system according to a first embodiment of the present invention;
Fig. 2 is a schematic illustration of an implant delivery device according to the first embodiment of the present invention;
Fig. 3 is a front view of Fig. 1;
Fig. 4 is an enlarged view of portion A of Fig. 3;
Fig. 5 is a schematic illustration of a master member according to the first embodiment of the present invention;
Fig. 6 is a schematic illustration of a slave member according to the first embodiment of the present invention;
Fig. 7 is a schematic illustration of a gear assembly according to the first embodiment of the present invention;
Fig. 8 is a front view of the gear assembly of Fig. 7;
Fig. 9 is a schematic illustration of a drive member according to the first embodiment of the present invention;
Fig. 10 is a schematic illustration of a coupling shaft according to the first embodiment of the present invention;
Fig. 11 is a schematic illustration of a housing according to the first embodiment of the present invention;
Fig. 12 is a cross-sectional view of Fig. 11 taken along direction B-B;
Fig. 13 is a schematic illustration of an implant delivery system according to a second embodiment of the present invention;
Fig. 14 is a front view of Fig. 13;
Fig. 15 is an enlarged view of portion C of Fig. 14; and
Fig. 16 is a schematic illustration of a housing according to the second embodiment of the present invention.

In these figures:
10-power conversion assembly; 11-master member; 12-slave member; 121-first internal bore; 122-second internal bore;
20-drive member; 200-special-shaped hole;
30-gear assembly; 31-first gear; 32-second gear;
40-coupling shaft; 400-special-shaped shaft section;
50-housing;
60-spacer;
70-catheter assembly; 71-first inner tube; 72-second inner tube; 73-third inner tube;
80-valve stent;
H-baseline.

### DETAILED DESCRIPTION

Objectives, advantages and features of the present invention will become more apparent upon reading the following more detailed description of the present invention with reference to the accompanying drawings. Note that the figures are provided in a very simplified form not necessarily drawn to exact scale and for the only purpose of facilitating easy and clear description of the embodiments. In addition, structures shown in the figures are usually part of actual structures. In particular, as the figures tend to have distinct emphases, they are often drawn to different scales.

As used herein, the singular forms "a", "an" and "the" include plural referents. As used herein, the term "or" is generally employed in the sense of "and/or", "several" of "at least one" and "at least two" of "two or more". Additionally, the use of the terms "first", "second" and "third" herein is intended for illustration only and is not to be construed as denoting or implying relative importance or as implicitly indicating the numerical number of the referenced item. Accordingly, defining an item with "first", "second" or "third" is an explicit or implicit indication of the presence of one or at least two of the items. The terms "one end" and "the other end", as well as "proximal end" and "distal end", are generally used to refer to opposing end portions including the opposing endpoints, rather than only to the endpoints. The terms "mounting", "coupling" and "connection" should be interpreted in a broad sense. For example, a connection may be a permanent, detachable or integral connection, a direct or indirect connection with one or more intervening media, or an internal communication or interaction between two elements. As used herein, when an element is referred to as being "disposed on" another element, this is generally intended to only mean that there is a connection, coupling, engagement or transmission relationship between the two elements, which may be either direct or indirect with one or more intervening elements, and should not be interpreted as indicating or implying a particular spatial position relationship between the two elements, i.e., the element may be located inside, outside, above, under, beside, or at any other location relative to the other element, unless the context clearly dictates otherwise. Those of ordinary skill in the art can understand the specific meanings of the above-mentioned terms herein, depending on their context.

The present invention provides an implant delivery device and an implant delivery system, which overcome the problem of inaccurate positioning of a valve stent during release associated with conventional delivery devices.

The proposed implant delivery device and implant delivery system will be described with reference to specific examples and the accompanying drawings.

It is to be noted that, as used herein, the term "proximal" generally refers to an end of a medical device closer to an operator who is operating it and the term "distal" generally refers to an end of the device that first enters the body of a patient, during normal operation of the device.

### Embodiment 1

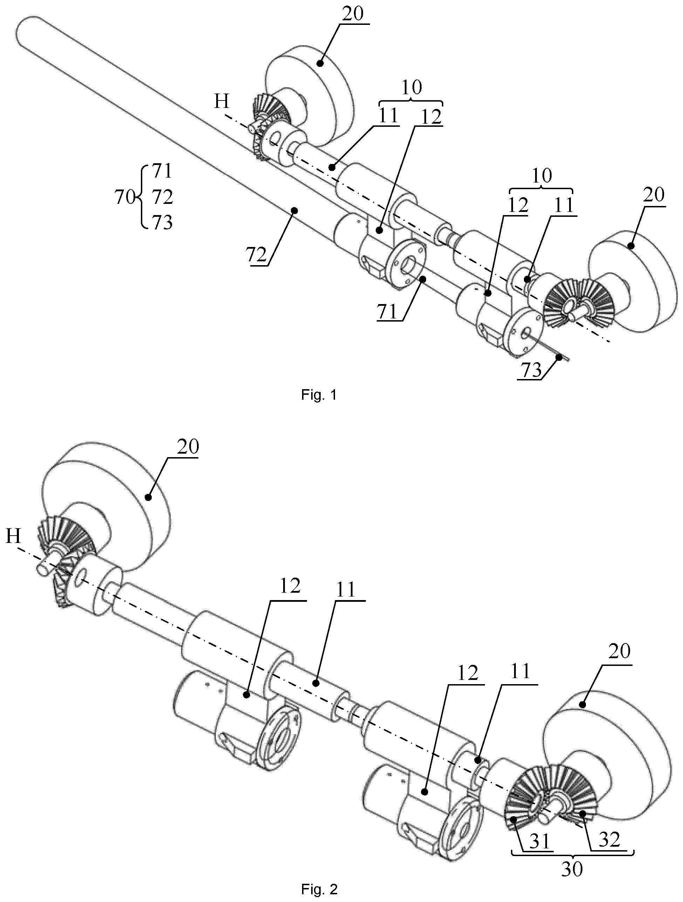

Figs. 1 to 12 show a first embodiment of the present invention.

Reference is made to Figs. 1 and 2. Fig. 1 is a schematic illustration of an implant delivery system according to the first embodiment of the present invention. Fig. 2 is a schematic illustration of an implant delivery device according to the first embodiment of the present invention. The present invention provides an implant delivery device applied to the implant delivery system. The implant delivery system includes the implant delivery device and a catheter assembly 70. The catheter assembly 70 includes a first inner tube 71 and a second inner tube 72. The first inner tube 71 is inserted in the second inner tube 72. The implant delivery device includes two power conversion assemblies 10 which are sequentially arranged in the direction of a baseline H, each power conversion assembly 10 includes a master member 11 and a slave member 12 in engagement with the master member 11. The slave member 12 of one of the two power conversion assemblies 10 is adapted for attachment to the first inner tube 71, and the slave member 12 of the other of the two power conversion assemblies 10 is adapted for attachment to the second inner tube 72. For example, in Fig. 1, the slave member 12 of the left power conversion assembly 10 is attached to the second inner tube 72, and the slave member 12 of the right power conversion assembly 10 is attached to the first inner tube 71. The power conversion assemblies 10 are configured to convert rotational motion of the master members 11 to linear motion of the respective slave members 12 in the direction of the baseline H. That is, the power conversion assemblies 10 are structures that convert rotational motion to linear motion. In this way, directions and velocities of movement of the slave members 12, and hence directions and velocities of relative movement of the first inner tube 71 and the second inner tube 72, can be controlled by adjusting movement of the respective master members 11 in the two power conversion assemblies 10. It would be appreciated that the direction of the baseline H is parallel to axial movements of the first inner tube 71 and the second inner tube 72 and that the baseline H is intended only for illustration of structural characteristics of the implant delivery device, rather than limitation.

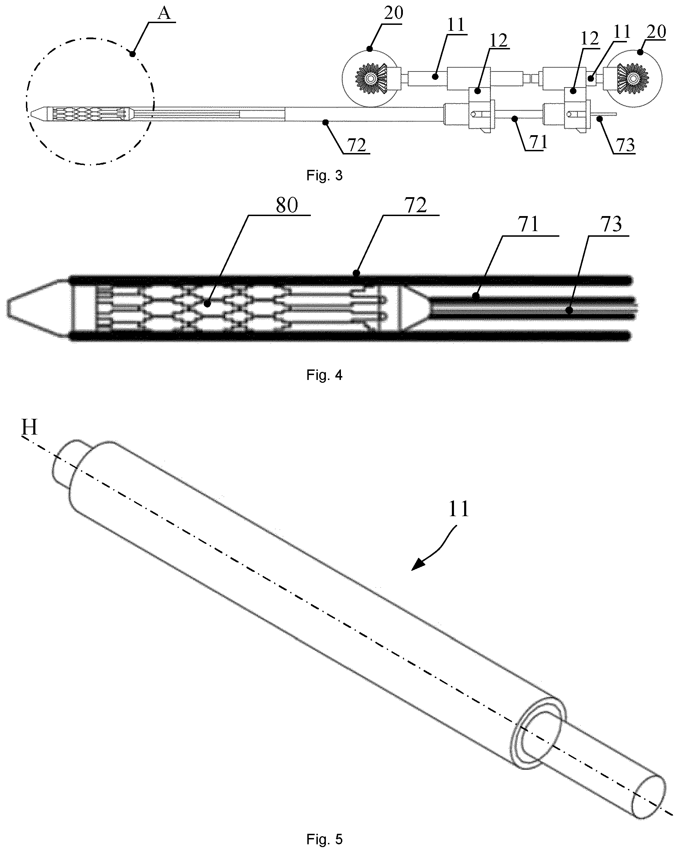

Reference is made to Fig. 3 and Fig. 4, Fig. 3 is a front view of Fig. 1, and Fig. 4 is an enlarged view of portion A of Fig. 3. The second inner tube 72 is adapted to restrict radial expansion of a valve stent 80, and the first inner tube 71 is adapted for connection with a proximal end of the valve stent 80. Specifically, the valve stent 80 can be considered as a roughly tubular structure. Moreover, the phrase "radial expansion" refers to radially outward expansion of the valve stent 80, and "radial contraction" refers to radially inward contraction of the valve stent 80. By "the second inner tube 72 is adapted to restrict radial expansion of a valve stent 80", it is intended to mean that the valve stent 80 is accommodated in the second inner tube 72 in a radially contracted configuration and thus restricted the valve stent 80 from radial expansion. The first inner tube 71 is coupled to the proximal end of the valve stent 80, and the first inner tube 71 is inserted within the second inner tube 72. With this arrangement, the first inner tube 71 and the second inner tube 72 can be moved relative to each other to release the valve stent 80 from the second inner tube 72 into the body of a patient. Once released, the valve stent 80 will expand radially. Alternatively, the first inner tube 71 and the second inner tube 72 can be moved relative to each other to retrieve the valve stent 80 back into the second inner tube 72 so that it is again accommodated within the second inner tube 72 in said radially contracted configuration.

As noted above, the implant delivery device includes two power conversion assemblies which are sequentially arranged in the direction of a baseline, each power conversion assembly includes a master member and a slave member in engagement with the master member. The slave member of one of the two power conversion assemblies is adapted for connection with the first inner tube, and the slave member of the other of the two power conversion assemblies is adapted for connection with the second inner tube. The power conversion assemblies are configured to convention rotational motion of the master members into linear motion of the slave members in the direction of the baseline. Controlling movement of the first inner tube 71 and the second inner tube 72 respectively with the two power conversion assemblies 10 enables desirable independent movement of the first inner tube 71 and the second inner tube 72 without mutual interference. Directions and velocities of movement of the slave members 12, and hence directions and velocities of relative movement of the first inner tube 71 and the second inner tube 72, can be controlled by adjusting movement of the respective master members 11 in the two power conversion assemblies 10. In this way, the valve stent 80 can be controlled so as to be released in two or more steps to facilitate precise release and positioning of the valve stent 80 without paravalvular leakage or regurgitation occurring in the patient. In addition, compared with the prior art, the use of the power conversion assemblies 10 according to the present invention decouples the power that drives movement of the first inner tube 71 and the second inner tube 72 from its original form that is provided by an external mechanism. This can avoid the device from having a complex structure and reduce its design and manufacturing costs.

It would be appreciated that the power conversion assemblies 10 are structures capable of converting rotational motion into linear motion. That is, they can convert rotational motion of the master members 11 into linear motion of the slave members 12. Directions of rotation of the master members 11 correspond to respective directions of translation of the slave members 12 and hence of the first inner tube 71/second inner tube 72. For example, referring to Fig. 1, in response to forward rotation of the master member 11 (clockwise rotation of the upper left master member 11 in the orientation of Fig. 1), the slave member 12 may translate toward the upper left, causing the second inner tube 72 to translate in the same direction. Moreover, in response to backward rotation of the master member 11 (counterclockwise rotation of the upper left master member 11 in the orientation of Fig. 1), the slave member 12 may translate toward the lower right, causing the second inner tube 72 to translate in the same direction. The same applies to the first inner tube 71 and, therefore, description thereof is not repeated herein. It is to be noted that the causal relationship between the direction of rotation of the master member 11 and the direction of translation of the slave member 12 for the first inner tube 71 may be identical or opposite to the causal relationship between the direction of rotation of the master member 11 and the direction of translation of the slave member 12 for the second inner tube 72.

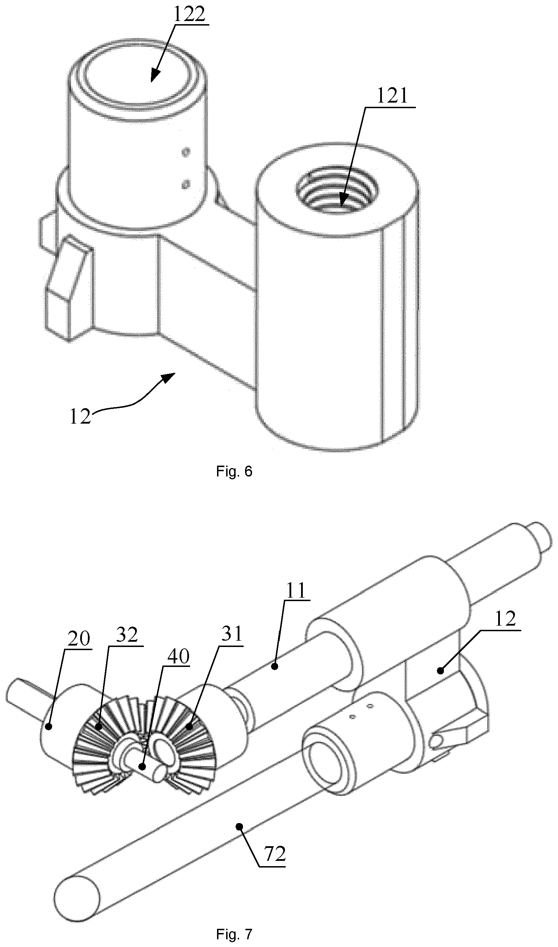

In a first exemplary implementation, referring to Fig. 5, Fig. 5 is a schematic illustration of one of the master members according to the first embodiment of the present invention, the master member 11 includes a screw, the screw extends in the direction of the baseline H, and the slave member 12 is threadedly engaged with an external thread section of the screw. That is, the power conversion assembly 10 functions like a ball screw, in which the master member 11 acts as a screw and the slave member 12 as a nut threadedly engaged with the screw. Rotational motion of the screw (i.e., rotation of the screw about its own center axis) is translated to linear motion of the nut. Further, with combined reference to Fig. 6, Fig. 6 is a schematic illustration of one of the slave members according to the first embodiment of the present invention, the slave member 12 defines a first internal bore 121 and a second internal bore 122, which both extend in the direction of the baseline H. The first internal bore 121 has an internal thread section engageable with the external thread section of the screw. The slave member 12 can be threadedly engaged with the master member 11 by the internal thread section of the first internal bore 121 and the external thread section of the screw. The second internal bore 122 of one of the two power conversion assemblies 10 is adapted for fixed attachment to the first inner tube 71, and the second internal bore 122 of the other of the two power conversion assemblies 10 is adapted for fixed attachment to the second inner tube 72. That is, the second internal bore 122 of one slave member 12 is adapted to fix the first inner tube 71, and the second internal bore 122 of the other slave member 12 is adapted to fix the second internal bore 122. The fixation may be accomplished, for example, by firmly bonding the inner tubes (the first inner tube 71 and the second inner tube 72) in the respective second internal bores 122.

In a second exemplary implementation (not shown), each power conversion assembly 10 is configured as a gear and rack assembly capable of power transmission. In this case, the master member 11 acts as a gear and the slave member 12 as a rack engageable with the gear. The rack extends in a direction parallel to the direction of the baseline H, and an axis of rotation of the gear is perpendicular to a plane defined by the rack. The gear can be rotated to cause advancement or retraction of the rack. That is, a direction of power transmission (forward or backward) of the rack can be controlled by controlling a direction of rotation of the gear so that the inner tube (the first inner tube 71 or the second inner tube 72) can be driven to move forward or backward.

In a third exemplary implementation (not shown), each power conversion assembly 10 is configured as a worm gear assembly capable of power transmission. In this case, the master member 11 acts as a worm and the slave member 12 as a worm gear engageable with the worm. The worm extends in the direction of the baseline H which is parallel to a plane defined by the worm gear, i.e., a center axis of the worm gear is perpendicular to the baseline H. A direction of rotation (forward or backward) of the worm gear can be controlled by controlling a direction of rotation of the worm (clockwise or counterclockwise rotation about its own center axis) so that the inner tube (the first inner tube 71 or the second inner tube 72) can be driven to move forward or backward.

It is to be noted that the power conversion assemblies 10 of this embodiment are not limited to the foregoing three implementations, any other mechanisms capable of translating rotational motion to linear motion can be suitably used as the power conversion assemblies 10 of this embodiment. Without departing from the scope of the present invention, the two power conversion assemblies 10 may be either identical or not. For example, each of them may be implemented as the ball screw shown in Figs. 1 and 2. Alternatively, one of them may be implemented as the ball screw, and the other as the worm gear assembly as described above.

Please refer to Figs. 1 and 2 for the manner of driving the master members 11. According to the present invention, the master members 11 may be driven to rotate by respective drive members 20 fixedly coupled to the respective master members 11. Specifically, the drive members 20 can drive the respective master members 11 to rotate, and directions of rotation of the master members 11 and hence directions of translation of the respective slave members 12 along the baseline H can be controlled by controlling directions of rotation of the respective drive members 20. The drive members 20 may be implemented as common handwheels. Rotation of the drive members 20 may be accomplished by external handles or motors coupled to the drive members 20, and forward or backward rotation of the motors may cause forward or backward translation of the respective slave members 12. Of course, those skilled in the art may select other method for controlling directions of rotation of the drive members 20 or of the master members 11, and further description in this regard is omitted herein.

Reference is made to Figs. 2 and 7. Fig. 7 is a schematic illustration of a gear assembly according to the first embodiment of the present invention. As shown, in one preferred implementation, the implant delivery device includes gear assemblies 30, each gear assembly 30 includes a first gear 31 and a second gear 32 in engagement with the first gear 31. The first gear 31 is coaxially coupled (or fixed) to a respective one of the master members 11. Here, by "coaxially", it is intended to mean that a center axis of the first gear 31 is coaxial with an axis of rotation of the master member 11. Additionally, the second gear 32 is coaxially coupled (or fixed) to a respective one of the drive members 20. Here, by "coaxially", it is intended to mean that a center axis of the second gear 32 is coincident with an axis of rotation of the drive member 20. The first gear 31 and the second gear 32 can work together to transmit a torque from the drive member 20 to the master member 11.

Further, when one slave member 12 drives the respective first inner tube 71/second inner tube 72 to move at a speed higher than a predetermined threshold, a transmission ratio of the second gear 32 to the first gear 31 is greater than 1. The transmission ratio of the second gear 32 to the first gear 31 may be configured within range M (1, +∞). When the slave member 12 drives the first inner tube 71/second inner tube 72 to move at a speed lower than or equal to the predetermined threshold, the transmission ratio of the second gear 32 to the first gear 31 is less than or equal to 1. The transmission ratio of the second gear 32 to the first gear 31 may be configured range N (0, 1]. When the slave member 12 drives the respective inner tube (the first inner tube 71 or the second inner tube 72) to move in synchronization therewith at a speed higher than the predetermined threshold, the movement of the slave member 12 and hence of the inner tube may be considered as "fast movement". In practical use, the fast movement of the inner tube may be correlated with the transmission ratio of the second gear 32 to the first gear 31. That is, when the transmission ratio of the second gear 32 to the first gear 31 is greater than 1, it can be considered that the inner tube is making fast movement. When the slave member 12 moves at a speed lower than or equal to the predetermined threshold, the movement of the slave member 12 and hence of the inner tube may be considered as "slow movement". In practical use, the slow movement of the inner tube may be correlated with the transmission ratio of the second gear 32 to the first gear 31. That is, when the transmission ratio of the second gear 32 to the first gear 31 is less than or equal to 1, it can be considered that the inner tube is making slow movement. Each master member 11 may be engaged with the respective slave member 12 in such a manner that a speed of movement of the slave member 12 may be controlled by adjusting a lead distance of the master member 11 with respect to the slave member 12. Here, the lead distance refers to a distance the slave member 12 moves (forward or backward) for every one revolution of the master member 11. Since each first gear 31 is coaxially coupled to the respective master member 11 and each second gear 32 is coaxially coupled to the respective drive member 20, and because each first gear 31 is engaged with the respective second gear 32, compared with the prior art, depending on a given application of the valve stent 80, the sizes of the first gears 31 and the second gears 32 may be selected so that their transmission ratio allows the drive members 20, the gear assemblies 30 and the master members 11 to work together to achieve the required fast or slow movement of each inner tube under the drive of the respective slave member 12. In this way, the slave members 12 can drive the respective inner tubes to make relative fast or slow movement as required by release of the valve stent 80 in the specific application to precisely satisfy an associated surgical need. The present embodiment is further described below with reference to three specific application scenarios. For ease of explanation, the "transmission ratio of the second gear 32 to the first gear 31" is referred to simply as the "transmission ratio".

In a first application scenario, the valve stent 80 can be positioned in the patient's body relatively easily and accurately, and it is therefore desired to quickly position the valve stent 80 and release it from the second inner tube 72 in order to result in surgical time savings. Accordingly, in this case, the transmission ratios of the two gear assemblies 30 may be both configured within range M, allowing the first inner tube 71 to make fast movement under the drive of one slave member 12 and the second inner tube 72 to also make fast movement under the drive of the other slave member 12. Thus, relative fast movement of the first inner tube 71 and the second inner tube 72 can be achieved, enabling quick positioning and release of the valve stent 80. It is to be noted that the two transmission ratios both within range M may be either equal to each other or not. In one exemplary implementation, for example, in each of the gear assemblies 30, the second gear 32 has 30 teeth, and the first gear 31 has 10 teeth. Therefore, their transmission ratios are both 3: 1. Accordingly, transmission ratios of the drive members 20 that are coupled to the respective second gears 32 to the master members 11 (the master members 11 are coaxially coupled to the respective first gears 31) are also configured to be 3: 1. For every one revolution of one drive member 20, the respective second gear 32 makes one revolution, the respective first gear 31 makes three revolutions and the respective master member 11 also makes three revolutions. As a result, the slave members 12 can drive fast movement of the respective inner tubes, enabling relatively fast relative movement of the first inner tube 71 and the second inner tube 72.

In a second application scenario, due to a complex anatomy, positioning and release of the valve stent 80 is relatively difficult. Therefore, a slow implantation process would be desirable, which involves slow release of the valve stent 80 allowing its simultaneous positioning. This can ensure that the valve stent 80 can be always adjusted in position throughout the release process until it is positioned at a desired site at the end of the process, resulting in a higher success rate of the surgical process for implanting the valve stent 80. Accordingly, in this case, the transmission ratios of the two gear assemblies 30 may be both configured within range N, allowing the first inner tube 71 to make slow movement under the drive of one slave member 12 and the second inner tube 72 to also make slow movement under the drive of the other slave member 12. Thus, relative slow movement of the first inner tube 71 and the second inner tube 72 can be achieved, enabling slow positioning and release of the valve stent 80. During release of the valve stent 80, it may be adjusted in position in real time depending on a condition of the valve stent 80 in the anatomy. It is to be noted that the two transmission ratios both within range N may be either equal to each other or not. In one exemplary implementation, for example, in each of the gear assemblies 30, the second gear 32 has 10 teeth, and the first gear 31 has 30 teeth. Therefore, their transmission ratios are both 1: 3. Accordingly, transmission ratios of the drive members 20 that are coupled to the respective second gears 32 to the master members 11 (the master members 11 are coaxially coupled to the respective first gears 31) are also configured to be 1: 3. For every three revolutions of one drive member 20, the respective second gear 32 makes three revolutions, the respective first gear 31 makes one revolution and the respective master member 11 also makes one revolution. As a result, the slave members 12 can drive slow movement of the respective inner tubes, enabling relatively slow relative movement of the first inner tube 71 and the second inner tube 72.

In a third application scenario, the valve stent 80 is suitable to be implanted in two steps including a first step requiring precise operation and accurate positioning due to high positioning difficulties (comparable to those encountered in the second scenario arising from a complex anatomy). After the positioning in the first step is complete, a second step starts for rapid release, which can result in surgical time savings. Accordingly, in this case, the transmission ratio of the gear assembly 30 for the second inner tube 72 may be configured within range N, and the transmission ratio of the gear assembly 30 for the first inner tube 71 may be configured within range M, allowing the valve stent 80 to be positioned at its distal end during slow release in the first step and then rapidly released in the second step following the positioning. In one exemplary implementation, for example, the transmission ratio of the gear assembly 30 for the second inner tube 72 is configured to be 1: 2 so that, for every two revolutions of the drive member 20, the second gear 32 makes two revolutions, the first gear 31 makes one revolution and the master member 11 also makes one revolution. As a result, the positioning of the valve stent 80 at the distal end can be accomplished in the first step during slow movement of the second inner tube 72 made under the drive of the slave member 12. Moreover, for instance, the transmission ratio of the gear assembly 30 for the first inner tube 71 is configured to be 2: 1 so that, for every one revolution of the drive member 20, the second gear 32 makes one revolution, the first gear 31 makes two revolutions and the master member 11 also makes two revolutions. As a result, after the positioning of the valve stent 80 is complete, the valve stent 80 can be rapidly released in the second step during fast movement of the first inner tube 71 made under the drive of the slave member 12.

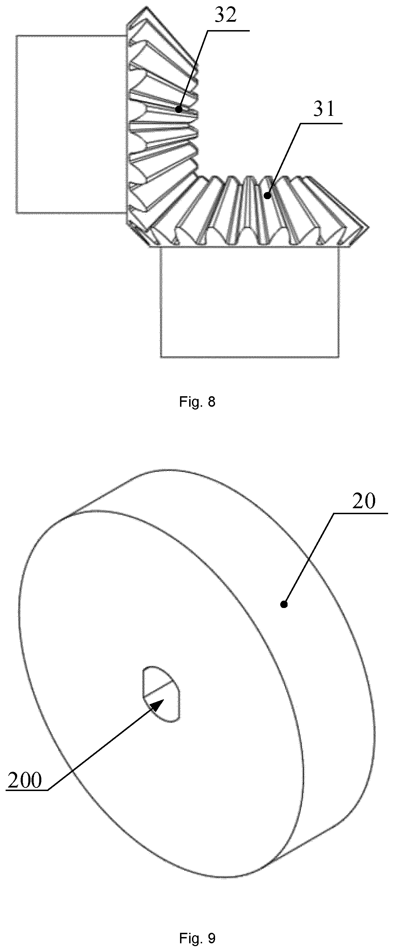

Preferably, referring to Fig. 8, Fig. 8 is a front view of one gear assembly of Fig. 7, both the first gear 31 and the second gear 32 are bevel gears, with an axis of rotation of the master member 11 being perpendicular to an axis of rotation of the drive member 20. The first gear 31 and the second gear 32 implemented as bevel gears can cooperate to transmit power. In specific implementations, the master member 11 may be implemented as a screw or worm. The power transmission accomplished with the bevel gears (i.e., the second gear 32 and the first gear 31) is smoother and steadier and produces less impact, vibration and noise.

It is to be noted that, in some other alternative implementations, power transmission between the drive member 20 and the master member 11 may be accomplished with more than two gears. That is, each gear assembly 30 may include more than two gears or other toothed components such as bevel gears or worm gear assemblies. All these would be easy to achieve for those skilled in the art.

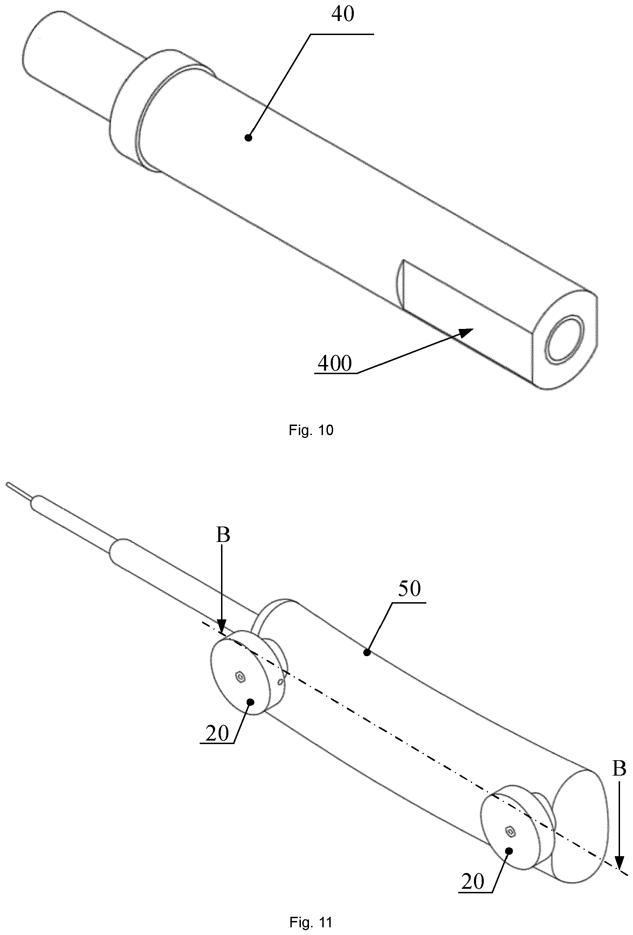

Reference is now made to Figs. 9 and 10. Fig. 9 is a schematic illustration of one drive member according to the first embodiment of the present invention, and Fig. 10 is a schematic illustration of a coupling shaft according to the first embodiment of the present invention. The coupling shaft 40 fixedly couples the drive member 20 to the second gear so that a center axis of the coupling shaft 40 coincides with an axis of rotation of the second gear. The drive member 20 defines a special-shaped hole 200, and the coupling shaft 40 has a special-shaped shaft section 400 matching the special-shaped hole 200 in shape. The special-shaped hole 200 and the special-shaped shaft section 400 can work together to accomplish the fixed coupling of the drive member 20 to the coupling shaft 40. The other end of the coupling shaft 40 is fixedly coupled to the second gear, allowing the drive member 20 to drive rotation of the second gear through the coupling shaft 40. It is to be noted that the special-shaped hole 200 refers to a non-circular hole such as a rectangular, trapezoidal or even irregularly-shaped bore. Accordingly, the special-shaped shaft section 400 may be a rectangular, trapezoidal or irregularly-shaped shaft section.

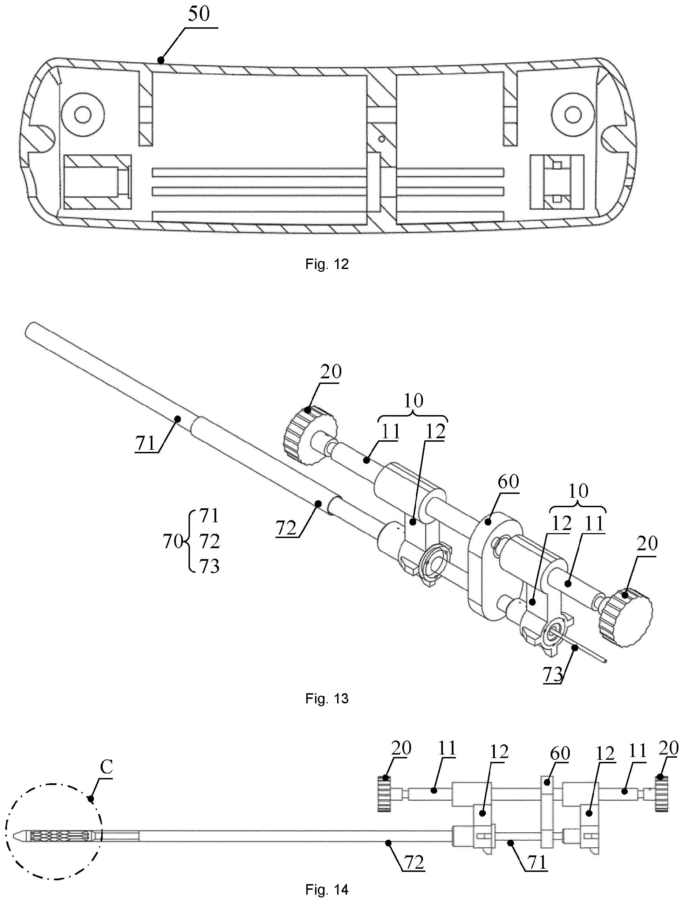

Reference is additionally made to Fig. 11, Fig. 11 is a schematic illustration of a housing according to the first embodiment of the present invention. The implant delivery device includes the housing 50, the housing 50 extends in the direction of the baseline H, the housing 50 generally assumes the shape of an elliptical cylinder. The power conversion assemblies 10 are accommodated inside the housing 50, and the components arranged within the housing 50 may include, but are not limited to, the gear assemblies and the coupling shafts 40. For ease of manipulation, the drive members 20 are disposed outside the housing 50. Fig. 12 is a cross-sectional view of Fig. 11 taken along direction B-B. As can be seen from Fig. 12, grooves and holes are formed in the housing 50 for accommodating the components including, but not limited to, the power conversion assemblies 10 and the coupling shafts 40.

On the basis of the implant delivery device discussed above, the present invention also provides an implant delivery system including: an implant delivery device as defined above; and a catheter assembly 70 including a first inner tube 71 and a second inner tube 72, the first inner tube 71 inserted in the second inner tube 72, the second inner tube 72 coupled to a slave member 12 in one of two power conversion assemblies 10, the second inner tube 72 adapted to restrict radial expansion of a valve stent 80, the first inner tube 71 coupled to a slave member 12 in the other of the two power conversion assemblies 10, the first inner tube 71 adapted for connection with a proximal end of the valve stent 80.

Optionally, referring to Figs. 1, 3 and 4, the catheter assembly 70 may include a third inner tube 73, the third inner tube 73 is inserted within the first inner tube 71. In fact, the third inner tube 73 may be adapted to accommodate a guide wire for insertion into the patient's body. The guide wire can provide guidance to the first inner tube 71 and the second inner tube 72 so that the valve stent 80 can be delivered over the guide wire to a lesion in the patient, particularly a designated site in a ventricular cavity of the patient.

### Embodiment 2

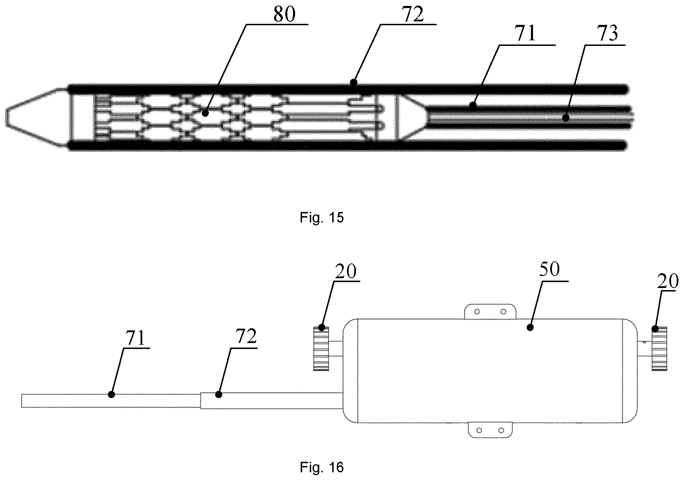

Figs. 13 to 16 show a second embodiment. Reference is made to Figs. 13 to 16, in conjunction with Figs. 1 to 12. Fig. 13 is a schematic illustration of an implant delivery system according to a second embodiment of the present invention. Fig. 14 is a front view of Fig. 13. Fig. 15 is an enlarged view of portion C of Fig. 14. Fig. 16 is a schematic illustration of a housing according to the second embodiment of the present invention. In the following, only differences of this embodiment from the first embodiment will be described, and description of any common feature that it shares with the first embodiment will not be repeated. For example, reference can be made to the description in connection with Figs. 3 and 4 for details of Figs. 14 and 15. Moreover, reference can be made to the description in connection with Fig. 11 substantially for details of Fig. 16.

Referring to Fig. 13, in this embodiment, the drive members 20 are coaxially coupled to respective master members 11, and axes of rotation of the master members 11 are parallel to a baseline H. That is, axes of rotation of the drive members 20 are coincident with the axes of rotation of the master members 11, the axes of rotation of the drive members 20 are parallel to the baseline H. Here, the master members 11 may be particularly implemented as the screws or worms discussed above. The drive members 20 are directly coupled to the master members 11 (e.g., through threaded engagement) so as to be able to drive the master members 11, the rotation direction of the drive members 20 is the same as the rotation direction of the master members 11, e.g., in clockwise or counterclockwise direction.

Optionally, the implant delivery device includes a spacer 60, the spacer 60 is perpendicular to the baseline H, the spacer 60 is disposed between the master members 11 in respective two power conversion assemblies 10, the spacer 60 is attached to ends of the master members 11 opposite to those thereof coupled to the drive members 20. In practice, the spacer 60 may be fixed in the housing 50 via a groove in the housing 50. The spacer 60 can not only fix the ends of the master members 11 opposite to those thereof coupled to the drive members 20 but can also prevent mutual interference between the two power conversion assemblies 10. The master members 11 may be attached to the spacer 60, for example, by inserting the ends of the master members 11 opposite to those thereof coupled to the drive members 20 in holes (not shown) formed in the spacer 60, which extend in the direction of the baseline H. A through hole (not shown) extending in the direction of the baseline H and adapted for passage of a second inner tube 72 therethrough may be also formed in the spacer 60.

It would be appreciated that since the implant delivery systems incorporate the respective implant delivery devices, they can provide the same benefits as the implant delivery devices. The foregoing description of the first and second embodiments focuses on the implant delivery devices, and the implant delivery systems further include the respective catheter assemblies 70, in addition to the implant delivery devices. Operation of the implant delivery systems and necessary auxiliary devices or components thereof will not be described in further detail herein, and those skilled in the art would know how the systems operate and how to configure the devices or components based on the common general knowledge in the art.

In summary, the present invention provides an implant delivery device and an implant delivery system. The implant delivery device is applied to a catheter assembly including a first inner tube and a second inner tube. The first inner tube is inserted in the second inner tube. The implant delivery device includes two power conversion assemblies which are sequentially arranged in the direction of a baseline, each of which includes a master member and a slave member in engagement with the master member. The slave member of one of the two power conversion assemblies is adapted for attachment to the first inner tube, and the slave member of the other of the two power conversion assemblies is adapted for attachment to the second inner tube. The power conversion assemblies are configured to convert rotational motion of the master members to linear motion of the slave members in the direction of the baseline. Controlling movement of the first and second inner tubes respectively with the two power conversion assemblies enables desirable independent movement of the first and second inner tubes without mutual interference. Moreover, relative movement of the first and second inner tubes can be controlled in a desired style to allow a stent to be released in two or more steps. This can facilitate precise release and positioning of the stent. Compared with the prior art, the use of the power conversion assemblies according to the present invention decouples the power that drives movement of the first and second inner tubes from its original form that is provided by an external mechanism. This can avoid the device from having a complex structure and reduce its design and manufacturing costs.

The description presented above is merely that of a few preferred embodiments of the present invention and is not intended to limit the scope thereof in any sense. Any and all changes and modifications made by those of ordinary skill in the art based on the above teachings fall within the scope as defined in the appended claims.

## Claims

1. An implant delivery device applied to a catheter assembly, the catheter assembly having a first inner tube and a second inner tube, the first inner tube inserted in the second inner tube, the implant delivery device comprising:
two power conversion assemblies which are sequentially arranged in a direction of a baseline, each of the power conversion assemblies comprising a master member and a slave member connected to the master member, the slave member of one of the two power conversion assemblies adapted for connection with the first inner tube, the slave member of the other of the two power conversion assemblies adapted for connection with the second inner tube, the power conversion assemblies configured to convert a rotational motion of the master members to a linear motion of the respective slave members in the direction of the baseline.

2. The implant delivery device according to claim 1, wherein the master member comprises a screw, the screw extending in the direction of the baseline, the slave member threadedly engaged with an external thread section of the screw.

3. The implant delivery device according to claim 2, wherein the slave member defines a first internal bore and a second internal bore, which both extend through the slave member in the direction of the baseline, the first internal bore having an internal thread section engageable with the external thread section of the screw, wherein the second internal bore of one of the two power conversion assemblies is adapted for fixed connection with the first inner tube, and the second internal bore of the other of the two power conversion assemblies is adapted for fixed connection with the second inner tube.

4. The implant delivery device according to claim 1, wherein the master member comprises a gear, the gear having an axis of rotation perpendicular to the baseline, wherein the slave member comprises a rack in engagement with the gear, and the rack extending in the direction of the baseline.

5. The implant delivery device according to claim 1, wherein the master member comprises a worm, the worm extending in the direction of the baseline, the slave member comprising a worm gear in engagement with the worm, and the worm having a center axis perpendicular to the baseline.

6. The implant delivery device according to claim 1, further comprising drive members, the drive members are connected to the respective master members, and the drive members adapted to drive the respective master members to rotate.

7. The implant delivery device according to claim 6, further comprising gear assemblies, each of the gear assemblies comprising a first gear and a second gear in engagement with the first gear, the first gear coaxially connected to the respective master member, the second gear coaxially connected to the respective drive member.

8. The implant delivery device according to claim 7, wherein both the first and second gears are bevel gears, wherein an axis of rotation of the master member is perpendicular to an axis of rotation of the drive member.

9. The implant delivery device according to claim 7, wherein when the slave member drives the corresponding first inner tube/second inner tube to move at a speed higher than or equal to a predetermined threshold, a transmission ratio of the second gear to the first gear is greater than 1, and wherein when the slave member drives the corresponding first inner tube/second inner tube to move at a speed lower than the predetermined threshold, the transmission ratio of the second gear to the first gear is smaller than or equal to 1.

10. The implant delivery device according to claim 7, wherein the second gear is fixedly connected to the drive member through a coupling shaft, the drive member is able to drive the second gear to rotate through the coupling shaft, the coupling shaft having a center axis coincident with an axis of rotation of the second gear, wherein the drive member defines a special-shaped hole, and the coupling shaft has a special-shaped shaft section matching the special-shaped hole in shape.

11. The implant delivery device according to claim 6, wherein the drive members are coaxially connected to the master members, each of the master members having an axis of rotation parallel to the baseline.

12. The implant delivery device according to claim 11, further comprising a spacer, the spacer oriented perpendicular to the baseline, the spacer disposed between the master members of the two power conversion assemblies, the spacer connected to ends of the master members opposite to those thereof connected to the drive members.

13. The implant delivery device according to claim 1, further comprising a housing, the housing extending in the direction of the baseline, wherein the power conversion assemblies are accommodated in the housing.

14. An implant delivery system, comprising:
the implant delivery device according to any one of claims 1 to 13; and
a catheter assembly comprising a first inner tube and a second inner tube, the first inner tube inserted in the second inner tube, the second inner tube connected to the slave member in one of the two power conversion assemblies, the second inner tube adapted to restrict radial expansion of a valve stent, the first inner tube connected to the slave member in the other of the two power conversion assemblies, the first inner tube adapted for connection with a proximal end of the valve stent.

15. The implant delivery system according to claim 14, wherein the catheter assembly further comprises a third inner tube, the third inner tube inserted in the first inner tube.
